# EUROPEAN PATENT APPLICATION

(11) **EP 3 466 436 A1**
(43) Date of publication of application: **10.04.2019**
(21) Application number: 17806425.9
(22) Date of filing: 19.05.2017
(51) Int. Cl.: A61K 36/185, A23L 33/10, A23L 33/105, A61K 31/352, A61K 36/752, A61K 45/00, A61P 25/00, A61P 25/16, A61P 25/28, A61P 43/00

(54) **PHARMACEUTICAL COMPOSITION AND FOOD**

(30) Priority: 01.06.2016 JP 2016109716
(71) Applicant: Sankyo Holdings Co., Ltd., Fuji-shi, Shizuoka 417-0061 (JP); Ohizumi, Yasushi, Sendai-shi, Miyagi 981-0943 (JP); Sendan Gakuen Educational Corporation, Sendai-shi, Miyagi 981-0943 (JP)
(72) Inventor: OHIZUMI Yasushi, Sendai-shi Miyagi 981-0943 (JP); KAJIMA Koji, Fuji-shi Shizuoka 416-0907 (JP); MARUYAMA Koji, Fuji-shi Shizuoka 417-0001 (JP)
(74) Representative: Strehl Schübel-Hopf & Partner
(86) International application number: PCT/JP2017/018913
(87) International publication number: WO 2017/208868

(57) **Abstract**

The purpose of the present invention is to provide a pharmaceutical composition and a food which have the effect of preventing central nervous system diseases and inhibiting the advancement thereof. This pharmaceutical composition, which is used to prevent and treat central nervous system diseases, is characterized by including: a dried product or an extracted liquid of Anredera cordifolia; and nobiletin, which is a polymethoxyflavonoid.

## Description

### [Technical Field]

The present invention relates to a pharmaceutical composition and a food and, in particular, relates to a pharmaceutical composition and a food each including Anredera cordifolia which has an action of ameliorating central nervous system diseases.

### [Background Art]

An increase in number of dementia patients in association with an ultra-aging society has become a serious society problem. Hereafter, the number of highly aged persons is forecasted to further increase, and requirements for pharmaceuticals and foods which prevent or ameliorate dementia are believed to be increased. As a plant-derived component which has an action of ameliorating memory impairment and the action mechanism of which is clearly understood, nobiletin derived from citrus peel has been known (for example, see PTL 1).

Nobiletin ameliorates memory impairments of various memory impairment model animals including Alzheimer's disease models. As the mechanism of the action thereof, it has been known that the protein kinase A (PKA)/(ERK)/cAMP (CRE)-binding prtein (CREB) intracellular signaling pathway is activated, the CRE-dependent transcription is enhanced, and the expression of memory and learning-related genes is enhanced (for example, see PTL 2).

On the other hand, attention has been paid on Anredera cordifolia which has an action of ameliorating hypertension and an action of inhibiting an increase in blood total cholesterol, neutral fat (triglyceride), and blood glucose level (for example, see PTL 3). Anredera cordifolia is a climbing perennial basella alba, is believed to be brought into Japan from Yunnan Province, China, and has been used as a food in some areas of Japan. In addition, although the stem of Anredera cordifolia extending in a vine shape has many propagules even over the ground, leaves, propagules, and root masses of Anredera cordifolia are also effectively usable, and a unique slimy component thereof is characteristic. In addition, Anredera cordifolia has been known as a plant having large contents of magnesium, copper, calcium, and zinc; however, Anredera cordifolia has not been researched nor investigated in the past.

### [Citation List]

### [Patent Literature]

[PTL 1]: Japanese Unexamined Patent Application Publication No. 2014-111664
[PTL 2]: Japanese Unexamined Patent Application Publication No. 2007-61028
[PTL 3]: Japanese Unexamined Patent Application Publication No. 2014-141438

### [Summary of Invention]

### [Technical Problem]

Heretofore, there has not been a definitive clinical drug having an effect of preventing dementia, a central nervous system disease, in particular, Alzheimer's diseases whose number of is approximately a half of that of dementia patients, and an effect of inhibiting the advancement of dementia. Hence, a pharmaceutical composition and a food having an effect of preventing central nervous system diseases and an effect of inhibiting the advancement thereof have been desired.

Accordingly, the present invention aims to provide a pharmaceutical composition and a food each of which has an effect of preventing central nervous system diseases and an effect of inhibiting the advancement thereof.

### [Solution to Problem]

A pharmaceutical composition according to the present invention is a pharmaceutical composition for preventing and treating central nervous system diseases and which is characterized by including a dried product or an extracted liquid of Anredera cordifolia and a polymethoxyflavonoid.

In addition, the pharmaceutical composition according to the present invention is characterized in that the polymethoxyflavonoid is nobiletin.

The pharmaceutical composition according to the present invention is characterized in that nobiletin is included in an extracted liquid of citrus plants.

The pharmaceutical composition according to the present invention is characterized in that the concentration of Anredera cordifolia in the extracted liquid of Anredera cordifolia is 50 to 100 µg/mL, and the concentration of nobiletin in the extracted liquid of citrus plants is 5 to 20 µg/mL.

The pharmaceutical composition according to the present invention is a food preventing and treating central nervous system diseases and which is characterized by including a dried product or an extracted liquid of Anredera cordifolia and a polymethoxyflavonoid. In addition, the pharmaceutical composition according to the present invention is characterized in that the polymethoxyflavonoid is nobiletin.

### [Advantageous Effects of Invention]

Since the dried product or the extracted liquid of Anredera cordifolia and the polymethoxyflavonoid are included, the pharmaceutical composition of the present invention has a CRE-dependent gene transcription activity, and hence, central nervous system diseases can be prevented, or the advancement thereof can be inhibited. In particular, when the polymethoxyflavonoid is nobiletin, the effect of preventing or ameliorating central nervous system diseases is significant.

In the pharmaceutical composition of the present invention, when the concentration of Anredera cordifolia in the extracted liquid of Anredera cordifolia is set to 50 to 100 µg/mL, and the concentration of nobiletin in the extracted liquid from citrus plants is set to 5 to 20 µg/mL, compared to the case in which a nobiletin solution is only used, the luciferase activity can be improved. In addition, since the luciferase activity is improved depending on the concentration of Anredera cordifolia, even if the concentration of Anredera cordifolia is higher than that described above, it is believed that the effect of improving the luciferase activity is generated.

When the nobiletin solution and Anredera cordifolia are added, compared to the case in which the nobiletin solution is only added, the CRE-dependent gene transcription activity is significantly increased higher than the theoretical value. Hence, when Anredera cordifolia and nobiletin are mixed together, a significant synergetic effect can be obtained.

In addition, as is the case described above, in the pharmaceutical composition of the present invention, when an extracted liquid of citrus plants including nobiletin and Anredera cordifolia are added, compared to the case in which the extracted liquid of citrus plants is only added, the CRE-dependent gene transcription activity is significantly increased higher than the theoretical value. Hence, when Anredera cordifolia and the extracted liquid of citrus plants including nobiletin are mixed together, a significant synergetic effect can be obtained.

In addition, even when the nobiletin concentration is low, the CRE-dependent gene transcription activity can be improved, and central nervous system diseases can be prevented, or the advancement thereof can be inhibited.

Furthermore, from an experiment on an effect of inhibiting the gene transcription activity by PD98059, the above pharmaceutical composition according to the present invention has an activating action for the gene transcription activity which is believed to be involved in the brain function.

The dried product or the extracted liquid of Anredera cordifolia may be applied to a food which is used to prevent and treat central nervous system diseases. In particular, when the dried product or the extracted liquid of Anredera cordifolia is applied to a food which includes nobiletin as the polymethoxyflavonoid, central nervous system diseases can be prevented, and the advancement thereof can be inhibited.

### [Brief Description of Drawings]

Fig. 1 is a graph showing the results of luciferase reporter assays obtained when an extracted liquid of Anredera cordifolia according to an embodiment of the present invention is added.
Fig. 2 is a graph showing the theoretical values and the results of luciferase reporter assays obtained when Anredera cordifolia with a nobiletin solution and Anredera cordifolia with an extracted liquid of peel of citrus plants (ponkan) according to the embodiment of the present invention are added.
Fig. 3 is a graph showing the results of luciferase reporter assays obtained when blending amounts of Anredera cordifolia and the extracted liquid of peel of citrus plants (ponkan) according to the embodiment of the present invention are changed.
Fig. 4 is a graph showing the results of an inhibitory effect caused by PD98059 obtained when the extracted liquid of Anredera cordifolia according to the embodiment of the present invention is added.

### [Description of Embodiments]

A pharmaceutical composition according to this embodiment includes a dried product or an extracted product of Anredera cordifolia and a polymethoxyflavonoid and is used to prevent and treat central nervous system diseases. In particular, the pharmaceutical composition according to this embodiment is used for Alzheimer's diseases and Parkinson's diseases.

In this embodiment nobiletin is used as the polymethoxyflavonoid.

[Sample Preparation] As Anredera cordifolia, Anredera cordifolia (Basella alba plant) produced in Fuji city, Shizuoka Prefecture was used and was extracted with methanol. An extracted Anredera cordifolia powder was dissolved in dimethyl sulfoxide (hereinafter, referred to as "DMSO") so that the concentrations of Anredera cordifolia in the extracted liquid were set to 50, 100, 200, 500, and 1,000 µg/mL.

The scientific name of Anredera cordifolia in Chinese is 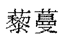 ( 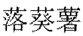 ), and Anredera cordifolia is called Anredera cordifolia (Tenore) Steenis in Latin, 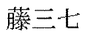 (teng san qi) as another name, and Boussingaultia gracilis Miersvar. pseudobaselloides Bai ley) in Latin.

As nobiletin, a product obtained from citrus plants by extraction was used (purity: 99%). Nobiletin was dissolved in DMSO so that the concentrations thereof were set to 2, 4, and 12 µg/mL.

A powder including nobiletin was extracted from citrus plants. As the citrus plants, Ohta Ponkan produced in Shimizu city, Shizuoka prefecture was used, and a product was extracted from ponkan's peel with methanol. A powder extracted from ponkan's peel was dissolved in DMSO so that the concentration of the ponkan' peel in the extracted liquid was set to 50 to 1,000 µg/mL.

In addition, the concentrations of nobiletin in the extracted liquids of the ponkan's peel at 50, 100, and 200 µg/mL were 0.5, 1.0, and 2.0 µg/mL, respectively.

In this embodiment, the following samples were used.
Sample 1: nobiletin solution (2 µg/mL) + extracted liquid of Anredera cordifolia (50 µg/mL);
Sample 2: nobiletin solution (2 µg/mL) + extracted liquid of Anredera cordifolia (100 µg/mL);
Sample 3: nobiletin solution (2 µg/mL) + extracted liquid of Anredera cordifolia (200 µg/mL);
Sample 4: nobiletin solution (4 µg/mL) + extracted liquid of Anredera cordifolia (50 µg/mL);
Sample 5: nobiletin solution (4 µg/mL) + extracted liquid of Anredera cordifolia (100 µg/mL);
Sample 6: nobiletin solution (4 µg/mL) + extracted liquid of Anredera cordifolia (200 µg/mL);
Sample 7: extracted liquid of ponkan's peel (50 µg/mL) + extracted liquid of Anredera cordifolia (50 µg/mL);
Sample 8: extracted liquid of ponkan's peel (50 µg/mL) + extracted liquid of Anredera cordifolia (100 µg/mL);
Sample 9: extracted liquid of ponkan's peel (50 µg/mL) + extracted liquid of Anredera cordifolia (200 µg/mL);
Sample 10: extracted liquid of ponkan's peel (100 µg/mL) + extracted liquid of Anredera cordifolia (50 µg/mL);
Sample 11: extracted liquid of ponkan's peel (100 µg/mL) + extracted liquid of Anredera cordifolia (100 µg/mL);
Sample 12: extracted liquid of ponkan's peel (100 µg/mL) + extracted liquid of Anredera cordifolia (200 µg/mL);
Sample 13: extracted liquid of ponkan's peel (200 µg/mL) + extracted liquid of Anredera cordifolia (50 µg/mL); Sample 14: extracted liquid of ponkan's peel (200 µg/mL) + extracted liquid of Anredera cordifolia (100 µg/mL).

[Luciferase Assay] In order to evaluate the gene transcription activity of each sample, the luciferase reporter assay was performed.

The luciferase assay is used to quantitatively analyze the transcription activity of a test material. In order to activate the transcription, various control sequences present in an upstream promoter of a gene are required.

In this embodiment, a plasmid was used in which a CRE sequence which was a control sequence activated by the PKA/ERK/CREB intracellular signaling and was present in an upstream promoter of a memory, and in which learning-related gene and a gene of a luciferase functioning as a luminescent enzyme were connected to each other.

When the test material activates the CRE-dependent transcription, the luciferase gene present at a downstream side is expressed, and the expression thereof is increased, so that a luciferase protein is increased. Accordingly, since luminescence is emitted by addition of a substrate, the absorbance thereof is measured, and hence, the transcription activity can be quantified.

PC12 cells (rat adrenal medulla pheochromocytoma derived cell line) were seeded in 96-well culture dish at a seeding density of 4.0×10⁴ cells/well. Subsequently, the plasmid was transfected in the PC12 cell, and the cultivation was performed in a medium to which each of the samples was dripped. As the medium, an α-modified Dulbecco's modified Eagle's medium supplemented with 1% serum (DMEM) was used.

After the PC12 cells were cultivated for a predetermined time, the culture medium was changed to a medium to which 100 µL of the sample was added, and the culture dish was incubated at 37°C in a 5%-CO₂ atmosphere for 5 hours, so that the luciferase reporter assay was performed.

In the individual samples, the final concentrations of nobiletin, Anredera cordifolia, and the extracted liquid of ponkan's peel with respect to the culture media are as follows.
Sample 1: nobiletin at 0.2 µg/mL and Anredera cordifolia at 5 µg/mL;
Sample 2: nobiletin at 0.2 µg/mL and Anredera cordifolia at 10 µg/mL;
Sample 3: nobiletin at 0.2 µg/mL and Anredera cordifolia at 20 µg/mL;
Sample 4: nobiletin at 0.4 µg/mL and Anredera cordifolia at 5 µg/mL;
Sample 5: nobiletin at 0.4 µg/mL and Anredera cordifolia at 10 µg/mL;
Sample 6: nobiletin at 0.4 µg/mL and Anredera cordifolia at 20 µg/mL;
Sample 7: extracted liquid of ponkan's peel at 5 µg/mL and Anredera cordifolia at 5 µg/mL;
Sample 8: extracted liquid of ponkan's peel at 5 µg/mL and Anredera cordifolia at 10 µg/mL;
Sample 9: extracted liquid of ponkan's peel at 5 µg/mL and Anredera cordifolia at 20 µg/mL;
Sample 10: extracted liquid of ponkan's peel at 10 µg/mL and Anredera cordifolia at 5 µg/mL;
Sample 11: extracted liquid of ponkan's peel at 10 µg/mL and Anredera cordifolia at 10 µg/mL;
Sample 12: extracted liquid of ponkan's peel at 10 µg/mL and Anredera cordifolia at 20 µg/mL;
Sample 13: extracted liquid of ponkan's peel at 20 µg/mL and Anredera cordifolia at 5 µg/mL; and
Sample 14: extracted liquid of ponkan's peel at 20 µg/mL and Anredera cordifolia at 10 µg/mL.

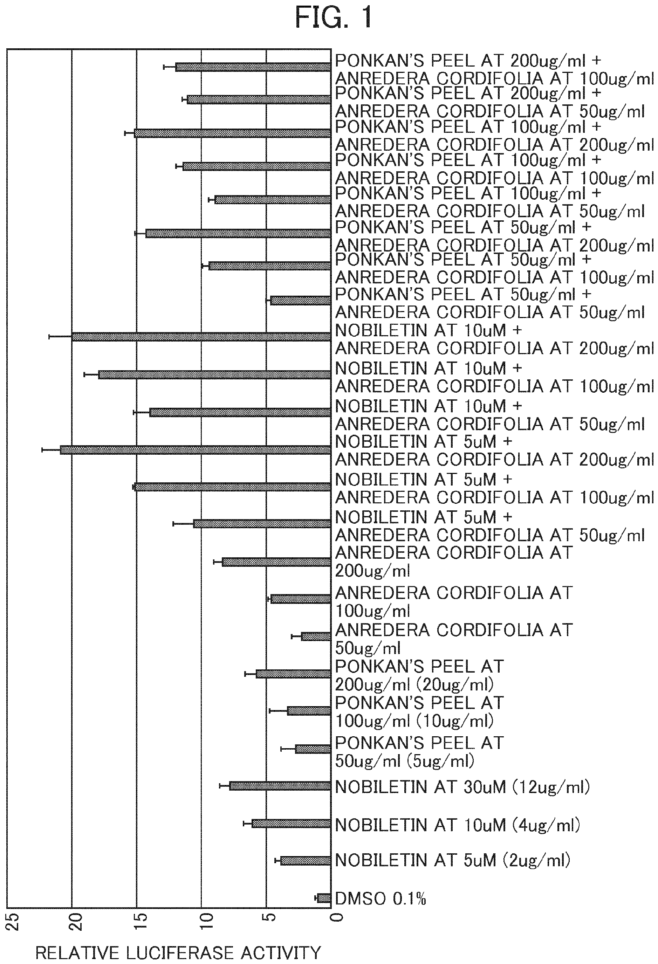

Fig. 1 shows the results of the luciferase reporter assay after each sample is added.

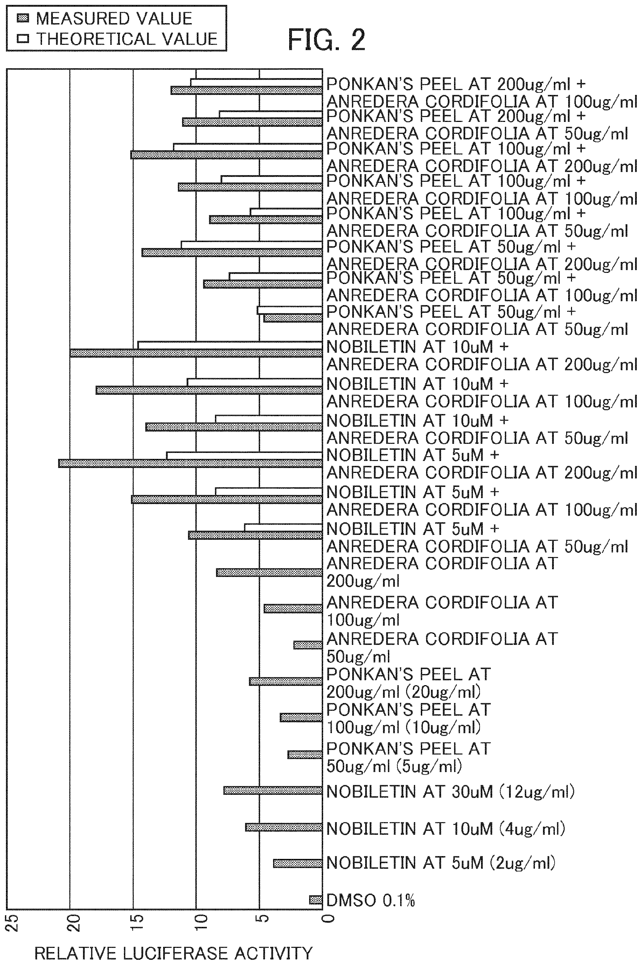

Furthermore, Fig. 2 is a graph in which the result of the luciferase activity of each sample and the theoretical value thereof are both shown. In this case, the theoretical value indicates the value which is obtained by adding the luciferase activities of samples to each other. For example, the theoretical value of Sample 1 is the value obtained by adding i) the value of the luciferase activity obtained when Anredera cordifolia at 50 µg/mL is only added to ii) the value of the luciferase activity obtained when a nobiletin solution at 2 µg/mL is only added.

Since the luciferase activity was obtained when Anredera cordifolia was only added, it was found that Anredera cordifolia had a transcription activity.

In addition, when the nobiletin solution and Anredera cordifolia were added to each other (Samples 1 to 6), compared to the case in which the nobiletin solution was only added, the luciferase activity was significantly increased, and hence, it was found that the value of each sample was larger than the theoretical value. From the results described above, it was found that when nobiletin and Anredera cordifolia were mixed together, a significant synergetic effect could be obtained.

In particular, in the cases of nobiletin at 2 µg/mL + Anredera cordifolia at 200 µg/mL (Sample 8) and nobiletin at 4 µg/mL + Anredera cordifolia at 100 µg/mL (Sample 5), the synergetic effect was significantly recognized.

When the extracted liquid of ponkan's peel and Anredera cordifolia were added (Samples 7 to 14), compared to the case in which the extracted liquid of ponkan's peel was only added, it was found that the luciferase activity was significantly increased and was larger than the theoretical value in almost all the samples. From the results described above, it was found that when the extracted liquid of ponkan's peel and Anredera cordifolia were mixed together, a significant synergetic effect could be recognized.

In particular, in the case in which ponkan's peel at 100 µg/mL + Anredera cordifolia at 50 µg/mL (Sample 10), a significant synergetic effect could be obtained.

When the cases in which ponkan's peel at 50 µg/mL + Anredera cordifolia at 50 µg/mL (Sample 7), ponkan's peel at 100 µg/mL + Anredera cordifolia at 100 µg/mL (Sample 11), and ponkan's peel at 200 µg/mL + Anredera cordifolia at 50 µg/mL (Sample 14) were compared to each other, the volume dependence was observed.

In addition, when ponkan's peel at 200 µg/mL (high concentration) and ponkan's peel at 50 µg/mL (low concentration) were each added, although the synergetic effect with Anredera cordifolia was low, in the case in which the extracted liquid of ponkan's peel at 100 µg/mL was added, it was found that the synergetic effect with Anredera cordifolia was high, and an enhancing action could be obtained.

Nobiletin expresses the luciferase activity at a concentration of 2 µg/mL. When the content of nobiletin in the extracted liquid of ponkan's peel was assumed to be 1%, the concentration of nobiletin in the extracted liquid of ponkan's peel at 200 µg/mL was 2 µg/mL. Accordingly, it is found that by the extracted liquid of ponkan's peel at 100 µg/mL (nobiletin: 1 µg/mL) which is a lower nobiletin concentration than the concentration at which the nobiletin solution expresses the luciferase activity, the synergetic effect can be obtained. From those described above, it is suggested that nobiletin has an enhancing action at 2 µg/mL or less.

Since the extracted liquids of ponkan's peel at 50, 100, and 200 µg/mL used in this embodiment were assumed to have nobiletin concentrations of 0.5, 1.0, and 2.0 µg/mL, respectively, in the case in which the nobiletin solution was added in combination with Anredera cordifolia, the minimum concentration at which the effect of the nobiletin solution was expressed was changed, and it was found that by the synergetic effect, the effect was expressed at a lower concentration of nobiletin.

In addition, in the solutions each prepared by mixing nobiletin to have a concentration of 0.2 µg/mL or 0.4 µg/mL (Samples 1 to 6), it was found that as the concentration of Anredera cordifolia was increased, the luciferase activity was synergistically improved. From the results described above, it is believed that the luciferase activity is improved depending on the concentration of Anredera cordifolia, and even if the concentration thereof is higher than the concentration of Anredera cordifolia used in this embodiment, a synergetic effect similar to that described above can be obtained.

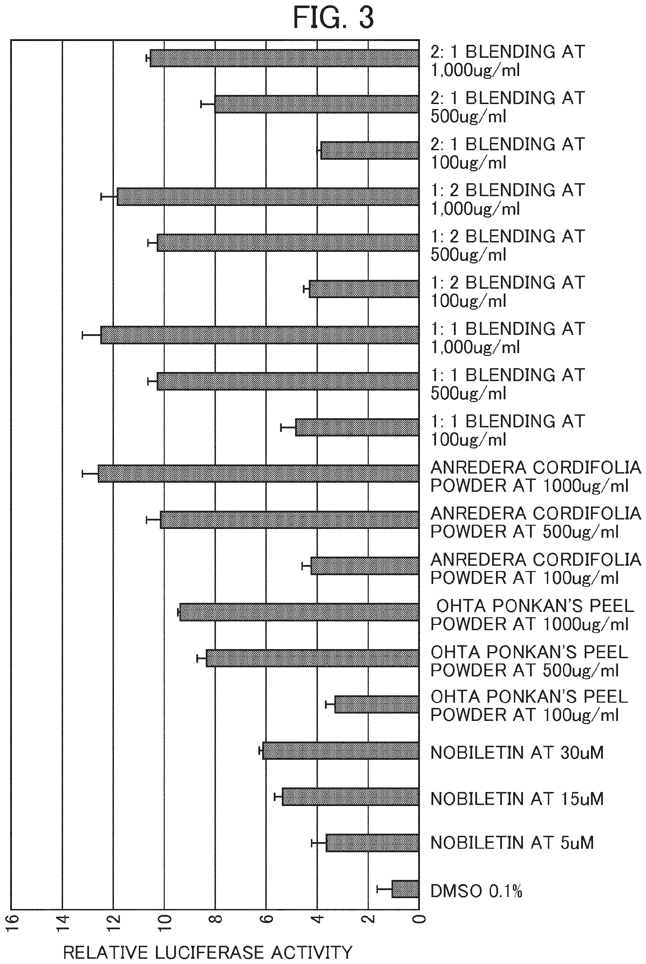

Fig. 3 shows the results of the luciferase report assay obtained when the blending amounts of Anredera cordifolia and the extracted liquid of ponkan's peel are changed.

When the extracted liquid of ponkan's peel at 500 µg/mL and Anredera cordifolia at 500 µg/mL were blended together at a ratio of 1: 1, and the extracted liquid of ponkan's peel at 500 µg/mL and Anredera cordifolia at 500 µg/mL were blended together at a ratio of 1: 2, it was found that a high luciferase activity (transcription activity) could be obtained.

### [Experimental Investigation on Inhibition Effect by PD98059]

Next, an experiment on inhibition effect by PD98059 was performed. By addition of PD98059 which is an inhibitor of transcription activity of genes, the transcription activity of genes is inhibited. When this inhibition activity is suppressed by the addition of this transcription activator, it is suggested that when the transcription activity of genes is maintained or increased, the gene transcription activity may be probably enhanced. It is believed that in the brain function, by administration of a material which activates the gene transcription activity, the brain function is maintained. Heretofore, it has been clearly understood from the research on nobiletin that as a mechanism of maintaining and ameliorating the brain function, the transcription activity of genes is involved, and the gene activity is enhanced and activated.

The investigation on the inhibition effect of the gene transcription by PD98059 was performed using Anredera cordifolia (dried product and extracted liquid) and Anredera cordifolia with the extracted liquid of ponkan's peel of this embodiment.

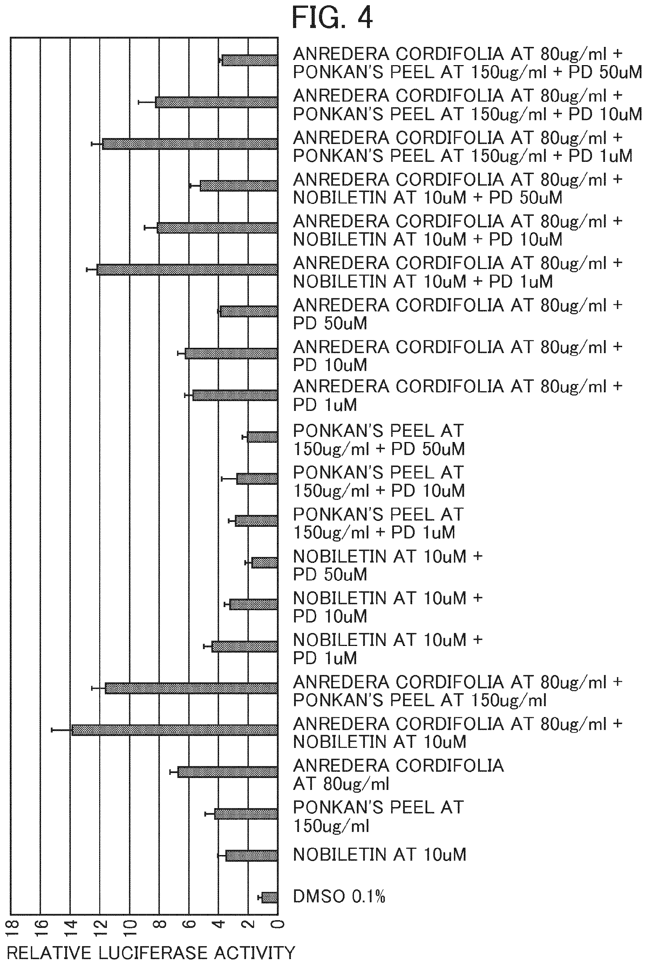

Fig. 4 shows the results of the inhibition effect by PD98059.

From those results, an activating action relating to the transcription activity of genes assumed to be involved in the brain function was recognized. Hence, it was found that Anredera cordifolia and Anredera cordifolia with the extracted liquid of ponkan's peel could be expected to have an effect of maintaining and ameliorating the brain function by a mechanism similar to that of nobiletin.

Hence, by performing gene transcription activation which relates to a long-term memory, the effect against central nervous system diseases (dementia, Parkinson's diseases, and the like) could be confirmed.

In addition, since Anredera cordifolia and ponkan's peel can be used as a food, those may be applied to foods to prevent or treat central nervous system diseases. Those foods each may be used either in a liquid or a solid form.

Heretofore, although this embodiment has thus been described, besides this embodiment, without departing from the scope of the present invention, the compositions described in the above embodiment may be appropriately selected or may be appropriately changed to have a different composition. For example, in this embodiment, although the case in which a methanol extracted liquid of Anredera cordifolia is used has been described by way of example, an extracted liquid obtained by extraction using another alcohol (such as butanol) may also be used. In addition, a dried product of Anredera cordifolia may also be used.

Furthermore, in this embodiment, as citrus plants, although ponkan is used, besides ponkan, for example, there may also be used calamondin, immature orange, citrus aurantium , Mediterranean mandarin, Dancy tangerine, Oobenimikan (Citrus tangerina), Kobenimikan (Citrus erythrosa), seedless Kishu, Fukuremikan (C tumida Hort.ex Tanaka), Kabuchii, Hirakishu, Citrus sunki, Cleopatra (Citrus reshni), Citrus leiocarpa, Citrus tardiva, Citrus wilsonii, Citrus unshiu, Citrus depressa, Citrus junos, Pomelo, Citrus tamurana, ponkan(Citrus poonensis), Citrus natsudaidai, navel orange, Citrus hassaku, Citrus iyo, and Kabosu (Citrus sphaerocarpa). In addition, in this embodiment, although nobiletin has been used as the polymethoxyflavonoid, besides the polymethoxyflavonoid, tangeretin, tetramethoxyflavone, pentamethoxyflavone, heptamethoxyflavone, or the like may also be used.

## Claims

1. A pharmaceutical composition for preventing and treating central nervous system diseases, comprising a dried product or an extracted liquid of Anredera cordifolia, and a polymethoxyflavonoid.

2. The pharmaceutical composition according to Claim 1, wherein the polymethoxyflavonoid is nobiletin.

3. The pharmaceutical composition according to Claim 2, wherein the nobiletin is included in an extracted liquid of citrus plants.

4. The pharmaceutical composition according to Claim 2 or 3, wherein a concentration of the Anredera cordifolia in the extracted liquid of Anredera cordifolia is 50 to 100 µg/mL, and a concentration of the nobiletin in the extracted liquid of citrus fruits is 5 to 20 µg/mL.

5. A food used for preventing and treating central nervous system diseases, comprising a dried product or an extracted liquid of Anredera cordifolia and a polymethoxyflavonoid.

6. The food according to Claim 5, wherein the polymethoxyflavonoid is nobiletin.
